# EUROPEAN PATENT APPLICATION

(11) **EP 2 270 057 A1**
(43) Date of publication of application: **05.01.2011**
(21) Application number: 09735480.7
(22) Date of filing: 24.04.2009
(51) Int. Cl.: C08F 2/44, A61F 13/49, A61F 13/53, C08F 6/00, C08K 3/00, C08K 5/05, C08K 5/09, C08L 33/02

(54) **WATER-ABSORBABLE POLYACRYLIC ACID (SALT) RESIN AND PROCESS FOR PRODUCTION THEREOF**

(30) Priority: 25.04.2008 JP 2008114946; 25.04.2008 JP 2008114947; 19.05.2008 JP 2008130617
(71) Applicant: Nippon Shokubai Co., Ltd., Osaka-shi, Osaka 541-0043 (JP)
(72) Inventor: FUJINO, Shinichi, Himeji-shi Hyogo 671-1152 (JP); NAGASAWA, Eri, Himeji-shi Hyogo 671-1131 (JP); ISHIZAKI, Kunihiko, Suita-shi Osaka 565-0873 (JP); KASUGA, Hiroto, Himeji-shi Hyogo 671-1154 (JP)
(74) Representative: HOFFMANN EITLE
(86) International application number: PCT/JP2009/001899
(87) International publication number: WO 2009/130915

(57) **Abstract**

In order to realize, while preventing gelatification of a monomer not yet to be polymerized, a water-absorbent resin which is more stable in color over time and has a significant enhancement in a problem concerning surface color and an excellent absorption property, a method for producing a polyacrylic acid-based water-absorbent resin includes: a preparation step in which a monomer is prepared from acrylic acid including hydroxyacetone in an amount not larger than 300 mass ppm; a polymerization step in which the monomer is polymerized so that a hydrogel is obtained; and a drying step in which the hydrogel is dried. Also, a method of the present invention for producing polyacrylic acid-based water-absorbent resin includes: a preparation step in which a monomer is prepared from acrylic acid; a polymerization step in which the monomer is polymerized in presence of phenol so that a hydrogel is obtained; and a drying step in which the hydrogel is dried.

## Description

### Technical Field

The present invention relates to a polyacrylic acid (salt)-based water-absorbent resin and a method for producing it. More specifically, the present invention relates to the following (i) and (ii): (i) a water-absorbent resin which is substantially white in color and of which a monomer stability at time of polymerization is excellent; and (ii) a method for producing the water-absorbent resin (i).

### Background Art

A water absorbent resin (SAP/Super Absorbent Polymer) is a water-swellable and water-insoluble polymeric gellant, which is widely and mainly used in disposable articles including (i) an absorbing article such as a disposable diaper, a sanitary napkin, and the like, (ii) an agricultural water retaining agent, and (iii) an industrial waterproofing agent. As a material for the water-absorbent resin, varieties of monomers and hydrophilic polymers have been proposed. Among them, a polyacrylic acid (salt)-based water-absorbent resin, which is formed by using acrylic acid and/or a salt thereof as a monomer, is widely used in industrial purposes due to its high water absorption performance.

A variety of properties (parameters) are known as water absorption properties desired in the water-absorbent resin. Examples of such properties encompass an absorbency without pressure, an absorbency against pressure, a water absorbing rate, a permeability potential without pressure, a permeability potential against pressure, an impact resistance, an anti-urine property, a fluidity, a gel strength, a particle size, and the like. They are basic physical properties, many of which are defined in EDANA (European Disposables and Nonwovens Associations), JIS (Japan Industrial Standard), and a parameter patent regarding a water-absorbent resin.

An acrylic acid (salt)-based monomer in the water-absorbent resin is easier to be polymerized and unstable. Due to such natures, the acrylic acid (salt)-based monomer easily forms a gel (polymer) even before being polymerized. The gel is formed in a small amount as a by-product and deposited in a tank and/or a pipe, particularly in polymerization carried out at a plant scale, polymerization started at high temperature, or polymerization of a monomer having a high concentration. Periodical removal of the gel or the like, however, causes a decrease in a productivity.

Possible countermeasures to partial polymerization of the monomer occurring prior to the polymerization include cooling of the monomer, increasing of a polymerization inhibitor in amount, and/or feeding of oxygen. However, such countermeasures cause a delay in the polymerization and, in some cases, cause deteriorations in the physical properties. Further, the increasing of a polymerization inhibitor in amount ends up with coloring of a water-absorbent resin (particularly, early-phase coloring which is described later).

An attempt to increase an amount of the water-absorbent resin in a diaper and to decrease an amount of a white pulp therein (i.e., an attempt to provide a diaper having a high concentration of SAP) has been made in order to realize a thinner diaper and/or a high-performance diaper. However, although a normal water-absorbent resin is a white powder at time of the early-phase coloring, the attempt may pose a problem that causes a decrease in whiteness of the diaper. The diaper suffering such decrease is deteriorated in a commercial value. A normal water-absorbent resin exists in a form of a white powder, even after being colored by early-phase coloring. However, the increasing of such water-absorbent resin in place of the white pulp may pose a problem that decreases whiteness of the diaper, thereby degrading a commercial value of the diaper. Also, there is another problem in that the water-absorbent resin, which is generally of the white powder, is changed in color over time. That is, the water-absorbent resin is known to be changed in color (in a range from yellow to brown) even after being shipped from a plant where it has been produced, e.g., when it stored and/or transported and even when it is used for a sanitary material. Thus, the water-absorbent resin is required to remain white in color, even in a case where an absorption article is stored for an extended period of time.

A variety of methods are proposed as countermeasures to the early-phase coloring of the water-absorbent resin or the change in the color of the water-absorbent resin over time. Examples of the method include: a method (see Patent Literatures 1 and 2) in which an organophosphate compound or a salt thereof is added to the water-absorbent resin; a method (see Patent Literature 3) in which a total amount of hydroquinone and benzoquinone in acrylic acid is controlled so as not to be larger than 0.2 mass ppm; a method (see Patent Literatures 4 and 14) in which an amount of a methoxyphenol compound in acrylic acid is controlled so as to be between 10 to 160 mass ppm; a method (see Patent Literatures 5 and 15) in which an inorganic reducing agent is added to the water-absorbent resin; a method (see Patent Literatures 6 through 8) in which organic carboxylic acid or a salt thereof is added to the water-absorbent resin; a method (see Patent Literature 9) in which polymerization is carried out by using tocopherol as a polymerization inhibitor; a method (see Patent Literature 10) in which polymerization is carried out by using steric barrier phenol; a method (see Patent Literatures 11 and 12) in which a metal chelating agent is used in production of a water-absorbent resin; and a method (see Patent Literature 13) in which an acrylic acid monomer and/or a salt thereof is polymerized by using hydroxyperoxide and a reducing agent, and treated by a silane coupling agent.

However, all of the methods pose problems. One of the problems is that the water-absorbent resin, which is fresh out of the plant, is made resistible to coloring by a sufficient. Another one of the problems regards control of polymerization reaction for the production of the water-absorbent resin. Still another one of the problems regards the deteriorations in the physical properties. An acrylic acid-based monomer, of which the water-absorbent resin is formed, is easy to be polymerized and unstable. Due to such natures, the acrylic acid-based monomer easily forms a partial gel (polymer) even before being polymerized.

That is, the countermeasures to prevent the coloring of the water-absorbent resin, such as decreasing of the polymerization inhibitor in amount or adding of a color preventing agent to the monomer, prevents the coloring of the water-absorbent resins by certain degrees (i.e., increases in a white index are obtained), while posing the problem that causes the deteriorations in the physical properties of the water-absorbent resin and the problem that causes the decrease in the stability of the monomer. Also, the countermeasures pose another problem, particularly in case of carrying out the polymerization at the plant scale (e.g., 1 Mt/hr), or in case of starting the polymerization at high temperature or carrying out the polymerization of the monomer having the high concentration. This is because in such polymerizations, the gel is formed in a small amount as a byproduct and deposited in the polymerization tank or the pipe, but the removal of the gel or the like causes the decrease in the productivity.

A water-absorbent resin for use in a diaper or the like is consumed and disposed in a large quantity. Regarding this, there has been a movement requiring that the water-absorbent resin be made from a nonfossil material (natural product) and be both sustainable and renewable. However, the water-absorbent resin made from a natural product is disadvantageous in terms of color (whiteness). Also, there has been proposed a water-absorbent resin which is made from acrylic acid (later described) prepared from a natural product such as glycerin. However, surprisingly, such water-absorbent resin is found to be disadvantageous in terms of whiteness, despite a fact that it is made from acrylic acid just like other types of water-absorbent resins. Patent Literature 16 proposes a method for producing a polymer, in which acrylic acid which is prepared by breaking down an organic compound by an enzyme is polymerized. Patent Literature 17 discloses a water-absorbent resin structure, (β1) the water-absorbent resin structure being an acrylic acid-based water-absorbent resin structure that includes 25 weight % or more of acrylic acid, and the water-absorbent resin structure being prepared by use of an acrylic acid monomer, 80 weight % or more of which acrylic acid monomer is obtained by a synthesis process whose initial step treats a nonfossil and renewable organic material, (β2) the water-absorbent resin structure having a biodegradability of 25 %, according to measurement carried out 28 days after the preparation of the water-absorbent resin structure by a ready biodegradability test in appendix V of a guide 67/548/EWG, and (β3) the water-absorbent resin structure having a CRC value not less than 20 g/g, according to measurement carried out in accordance with ERT441.2-02. Also, both of Patent Literatures 17 and 18 disclose a polymer structure, which is a partially neutralized acrylic acid-based water-absorbent polymer structure and has a stability coefficient not lower than 80 %.
Patent Literature 1
   Japanese Patent Application Publication, Tokukaihei, No. 5-86251 A
Patent Literature 2
   Japanese Patent Application Publication, Tokukaihei, No. 1-275661 A
Patent Literature 3
   U.S. Patent No. 6444744
Patent Literature 4
   U.S. Patent No. 7049366
Patent Literature 5
   International Publication No. 2000/55245
Patent Literature 6
   Japanese Patent Application Publication, Tokukai, No. 2000-327926 A
Patent Literature 7
   Japanese Patent Application Publication, Tokukai, No. 2003-52742 A
Patent Literature 8
   Japanese Patent Application Publication, Tokukai, No. 2005-186016 A
Patent Literature 9
   International Publication No. 2003/53482
Patent Literature 10
   International Publication No. 2005/54356
Patent Literature 11
   U.S. Patent Application Publication No. 2005/0085604
Patent Literature 12
   Japanese Patent Application Publication, Tokukai, No. 2003-206381 A
Patent Literature 13
   Japanese Patent Application Publication, Tokukaihei, No. 4-331205 A
Patent Literature 14
   International Publication No. 2007/028748
Patent Literature 15
   U.S. Patent Application Publication No. 2006/0074160
Patent Literature 16
   International Publication No. 2006/092271
Patent Literature 17
   International Publication No. 2006/092272
Patent Literature 18
   International Publication No. 2008/023040

### Summary of Invention

The present invention is made in view of conventional problems described earlier (which include (i) partial gelatification of a monomer not having been subjected to polymerization and (ii) coloring of a water-absorbent resin), and an object of the present invention is to prevent such partial gelatification of the monomer, and to provide a method for producing a water-absorbent resin more stable in color over time. A more preferable object of the present invention regards a water-absorbent resin which, in large amount, is used in article such as a diaper or the like. The more preferable object of the present invention is to provide the water-absorbent resin which is sustainable and renewable and is excellent in whiteness. The present invention is also made in view of other conventional problems described earlier (which include (iii) coloring of the water-absorbent resin, and (iv) deterioration in a physical property and a decrease in productivity (decrease in a stability of a monomer) which arise from countermeasures for preventing the coloring of the water-absorbent resin), and still another object of the present invention is to provide a water-absorbent resin which has a significant enhancement in a problem concerning surface coloring and is excellent in a water absorption property, and to provide a method for producing the water-absorbent resin without causing either the deterioration in the physical property or the decrease in the productivity.

The inventors of the present invention had conducted diligent studies in efforts of attaining the objects, and found the followings by studying an effect of hydroxyacetone, which was a minor component in acrylic acid. Namely, hydroxyacetone caused the decrease in the stability of the monomer not having been subjected to polymerization, and was a cause of the change in color of the water-absorbent resin over time. Furthermore, the water-absorbent resin made from acrylic acid derived from natural glycerin is easier to be colored, since such acrylic acid contains a relatively large amount of hydroxyacetone.

Considering the above, in order to attain the objects, the present invention provides a method for producing a polyacrylic acid (salt)-based water-absorbent resin, the method including: a preparation step in which a monomer is prepared from acrylic acid (salt) including hydroxyacetone in an amount not larger than 300 mass ppm; a polymerization step in which the monomer is polymerized so that a hydrogel is obtained; and a drying step in which the hydrogel is dried.

Also, in order to attain the objects, the present invention provides a water-absorbent resin, which (i) is a polyacrylic acid-based water-absorbent resin of which polyacrylic acid is derived from natural glycerin and (ii) has YI ≤ 30 after being subjected to a color acceleration test.

Also, in order to attain the object, the present invention provides a method for producing a polyacrylic acid (salt)-based water-absorbent resin, the method including: a preparation step in which a monomer is prepared from acrylic acid (salt); a polymerization step in which the monomer is polymerized in the presence of phenol so that a hydrogel is obtained; and a drying step in which the hydrogel is dried. In addition, the present invention provides a polyacrylic acid (salt)-based water-absorbent resin including phenol.

This makes it possible to realize an increase in whiteness (reductions in a YI value and a b value as well as an increase in a WB value) of the water-absorbent resin, while securing the water absorption property and the stability of the monomer. This makes it possible to realize an absorption article such as a diaper, an absorbent material, or the like, each of which gives a very clean impression, while preventing itself from either allowing a leakage or giving slime feeling and wet feeling.

For a fuller understanding of the nature and advantages of the invention, reference should be made to the ensuing detailed description taken in conjunction with the accompanying drawings.

### Description of Embodiments

For a fuller understanding of the nature and advantages of the invention, reference should be made to the ensuing detailed description taken in conjunction with the accompanying drawings. The following description discusses the present invention in more detail.

### (1) Definition of Terms

### (a) "Water-Absorbent Resin"

A water-absorbent resin is a water-swellable and water-insoluble polymer gellant. A water absorbency (CRC) of the water-absorbent resin, which is set forth in ERT 441.2-02, is at least 5 g/g or more, preferably between 10 to 100 g/g, and more preferably between 20 to 80 g/g. Extractables of a water-absorbent resin, which are set forth in ERT 470.2-02, are at least between 0 to 50 weight % or lower, preferably between 0 to 30 weight %, more preferably between 0 to 20 weight %, and particularly preferably between 0 to 10 weight %.

The water-absorbent resin is not limited exclusively to a polymer. Instead, it can be a water-absorbent resin composition matter in which a polymer includes, therein, on a surface thereof, in other parts thereof, an additive agent (later described) in an amount which does not cause any harm to the quality of the water-absorbent resin described earlier. That is, in the present invention, the water-absorbent resin composition matter can also be referred to as the water-absorbent resin. Of an entire amount of the water-absorbent resin, an amount of a polyacrylic acid (salt)-based water-absorbent resin is preferably between 70 to 99.9 mass %, more preferably between 80 to 99.7 mass %, and still preferably between 90 to 99.5 mass %. Another component than the polyacrylic acid (salt)-based water-absorbent resin is preferably water, in consideration of a water absorption speed or an impact resistance of a powder (particle). The additive agent (later described) may be included if necessary.

### (b) "Polyacrylic Acid (Salt)"

Polyacrylic acid (salt) is a polymer including mainly acrylic acid (salt) as a repeat unit. Acrylic acid (salt), as a monomer excluding a cross-linking agent, is included in an amount at least between 50 to 100 mol %, preferably between 70 to 100 mol %, more preferably between 90 to 100 mol %, and most preferably of substantially 100 mol %. An acrylic acid salt as a polymer includes at least a water-soluble salt, preferably a monovalent salt, more preferably one or more of an acrylic metal salt and an ammonium salt, particularly preferably an alkali metal salt, and still preferably a sodium salt.

In the present invention, unless otherwise noted, polyacrylic acid is not limited to unneutralized polyacrylic acid. Alternatively, polyacrylic acid can be a polyacrylic acid salt.

### (c) "Early-Phase Coloring of Water-Absorbent Resin"

This is a color of a water-absorbent resin having just produced at a plant or that of a water-absorbent resin having just delivered to a customer. Generally, early-phase coloring (L value, a value, b value, YI value, WB value, and the like) of the water-absorbent resin is determined by color control, which is carried out at the plant before the water-absorbent resin is delivered to the customer.

### (d) "Coloring of Water-Absorbent Resin Over Time"

This is a phenomenon in which the water-absorbent resin, which has not been put in application or swollen, is gradually changed in color (usually, into yellow or brown) when being stored for an extended period of time or when being distributed in a market. For example, a water-absorbent resin in an unused diaper is changed in color, and this may possibly cause a degradation in a commercial value of the unused diaper. The coloring of the water-absorbent resin over time is a problem which occurs at room temperature over an extended span of time ranging from months to years. For this, it is possible to study the problem by carrying out a color acceleration test (high-temperature and high-humidity condition) later described.

### (e) "EDANA" and "ERT"

EDANA is an abbreviation for European Disposables and Nonwovens Associations. The following description discusses a measuring method (ERT/EDANA Recommended Test Method) of a water-absorbent resin, which is also a standard measuring method of a water-absorbent resin in Europe (and substantially in the entire world). For details of the measuring method, see an original text of ERT (revised in 2002), which is a publicly known literature. Note that in the description below, a number in parenthesis indicates a page number of the original English text of ERT.

### (f) "CRC" (ERT 441. 2-02)

CRC stands for a centrifugal retention capacity. CRC is an absorbency (unit: g/g) at which a water-absorbent resin, which is allowed to be freely swollen in a 0.9 % saline solution for 30 minutes and drained by centrifugation, absorbs the 0.9 % saline solution and then drained.

### (g) "AAP" (ERT 442. 2-02)

AAP stands for an absorbency against pressure. AAP is an absorbency (unit; g/g) at which a water-absorbent resin, which is placed under a pressure of 21g/cm², absorbs a 0.9 % saline solution for an hour. The pressure can be changed to 50g/cm² or the like, as needed.

### (h) "Extractables" (ERT 470. 2-02)

Extractables are soluble contents. The extractables are of a value determined through steps of adding 1 g of a water-absorbent resin in 200 g of a 0.9 % saline solution, stirring a mixture thereof for 16 hours, and measuring an amount of a polymer dissolved in 200 g of the 0.9 saline solution by pH titration.

### (i) "FSC" (ERT 420. 2-02)

FSC stands for a free swell capacity. FSC is an absorbency at which a water-absorbent resin, draining of which is not carried out by centrifugation, absorbs a 0.9% saline solution.

### (j) "Residual Monomer (ERT 410. 2-02)"

A residual monomer is determined by measuring, by liquid chromatography, an amount of a residual monomer dissolved into a 0.9% saline solution.

### (k) "Particle Size Distribution (ERT 420. 2-02)"

A particle size distribution is determined by carrying out sieve classification.

### (1) Measurement of Water-Absorbent Resin in Other Aspects Set Forth in EDANA (set forth in 2002).

### "pH" (ERT 400. 2-02)

This is pH of a water-absorbent resin.

### "Moisture Content" (ERT 430. 2-2)

This is a moisture content in a water-absorbent resin.

### "Flow Rate" (ERT 450. 2-02)

This is a flow rate of a water-absorbent resin powder.

### "Density" (ERT 460. 2-02)

This is a bulk specific gravity of a water-absorbent resin.

### "Respirable Particles". (ERT 480. 2-02)

### "DUST" (ERT 490. 2-02).

### (2) Monomer (Excluding Cross-Linking Agent)

In the present invention, a monomer is formed mainly of acrylic acid or a salt thereof, and in consideration of a water absorption property, a polymer formed of the monomer is preferable to have a neutralized acid radical. Neutralization of a acid radical of the polymer is carried out at a rate between 10 to 100 mol %, preferably between 30 to 95 mol %, more preferably between 50 to 90 mol %, and still preferably between 60 to 80 mol %. It is optional to carry out the neutralization of the acid radical with respect to the polymer (hydrate gel) after polymerization or with respect to the monomer prior to the polymerization. However, in consideration of physical properties, an improvement in AAP, or the like, it is preferable to carry out the neutralization of the acid radical with respect to the monomers. Thus, in the present invention, the monomer is a partially neutralized salt of acrylic acid. A water-absorbent resin of the present invention is a polyacrylic acid salt-based water-absorbent resin of which an acid radical has been neutralized at any of the rates, or is a partially-neutralized polyacrylic acid salt-based water-absorbent resin of which an acid radical has been neutralized at any of the rates. The term "acrylic acid (salt)" in the Specification of the subject application means acrylic acid and/or a salt thereof.

The present invention can use a hydrophilic unsaturated monomer or a hydrophobic unsaturated monomer other than acrylic acid (salt). Examples of a monomer usable in the present invention encompass methacrylic acid, (anhydrous) maleic acid, 2-(meth)acrylamide-2-methylpropanesulfonic, (meth)acryloxyalkane sulfonic acid, N-vinyl-2-pyrrolidone, N-vinylacetamide, (meth)acrylamide, N-isopropyl(meth)acrylamide, N,N-dimethyl(meth)acrylamide, 2-hydraxyethyl(meth)acrylate, methoxypolyethyleneglycol(meth)acrylate, polyethyleneglycol(meth)acrylate, stearylacrylate, and salts thereof.

### (3) Cross-Linking Agent (Internal Cross-Linking Agent)

In the present invention, it is particularly preferable, in view of water absorption property, to use a cross-linking agent (also referred to as internal cross-linking agent). In consideration of physical properties, an amount of the cross-linking agent to the monomer excluding the cross-linking agent is between 0.001 to 5 mol %, preferably between 0.005 to 2 mol %, further preferably between 0.01 to 1 mol %, and particularly preferably between 0.03 to 0.5 mol %.

Examples of the cross-linking agent usable in the present invention encompass one or more of a polymerizable cross-linking agent (which is polymerizable with acrylic acid), a reactive cross-linking agent (which is reactive to a carboxyl group), and a cross-linking agent including the cross-linking agents. Concrete examples of the polymerizable cross-linking agent encompass a compound having at least two polymerizable double bonds within a molecule, such as a N.N'-methylenebisacrylamide, (poly)ethylene glycol di(meth)acrylate, (polyoxyethylene) trimethylol propanetri(meth)acrylate, poly(meth)allyloxyalkane or the like. Examples of the reactive cross-linking agent encompass: a covalent cross-linking agent such as polyglycidyl ether (such as ethyleneglycol diglycidyl ether or the like), polyalcohol (such as propanediol, glycerine, sorbitol, or the like), or the like; and an ionic bonding cross-linking agent, which is a polyvalent metal compound such as aluminum. In view of a water absorption property, it is preferable to use the polymerizable cross-linking agent (which is polymerizable with acrylic acid) out of the above cross-linking agents. Particularly, it is preferable to use an acrylate-based polymerizable cross-linking agent, an aryl-based polymerizable cross-linking agent, and an acrylamide-based polymerizable cross-linking agent.

### (Neutralizing Salt)

A base material for use in neutralization of acrylic acid is preferably: a hydroxide of alkali metal such as sodium hydroxide, potassium hydroxide, lithium hydroxide, or the like; and a monovalent base of (hydrogen) carbonate or the like, such as sodium (hydrogen) carbonate, potassium (hydrogen) carbonate, or the like. Examples of a preferred condition under which the neutralization of acrylic acid is carried out or the like are taught in International Publication No. 2006/522181. These examples are applicable to the present invention. Temperature at which the neutralization of acrylic acid is carried out is determined as appropriately in a range between 10 to 100 °C or in a range between 30 to 90 °C. It is optional to carry out the neutralization of acrylic acid, which uses any of the monovalent bases, with respect to acrylic acid prior to polymerization or with respect to polyacrylic acid after polymerization. However, carrying out of the neutralization of acrylic acid with respect to polyacrylic acid, which is non-neutralized or low neutralized (neutralization rate lower than approximately 30 mol %), is time consuming and may be disadvantages in terms of physical properties such as improvement in AAP and the like. Therefore, in consideration of the physical properties, it is preferable to carry out the neutralization of acrylic acid prior to polymerization, so as to achieve partial neutralization of acrylic acid and prepare acrylic acid thus partially neutralized as a monomer.

### (5) Amount of Fe

In the present invention, it is preferable to prepare the monomer in such a manner that the monomer includes iron in a small amount or so that the monomer does not include iron at all. The preparation of the monomer includes a step in which an amount of iron (Fe₂O₃ basis) in the monomer is controlled so as to be between 0 to 10 mass ppm, preferably between 0 to 5 mass ppm, more preferably greater than 0 but lower than 5 mass ppm, further preferably between 0.001 to 5 mass ppm, particularly preferably between 0.001 to 4 mass %, and most preferably between 0.005 to 3 mass ppm, still most preferably between 0.05 between 1 mass ppm, and particularly most preferably between 0.06 to 0.5 mass ppm. In the step of the preparation of the monomer, this is carried out by carrying out control of a purity of an alkali metal salt, which is for use in the neutralization as a source of iron.

The following methods (a) and (b) are concrete examples of methods for carrying out the preparation of the monomer.

Method (a); a method (a) carries out the preparation of the monomer by steps of: using a base material (e.g., a hydroxide of alkali metal) selected from commercially available base materials and including iron in an amount between 0.01 to 10 mass ppm; and preparing the base material in such a manner that the base material contains 0.001 to 5 of Fe in a monomer.

Method (b); a method (b) carries out the preparation of the monomer by steps of: carrying out, with respect to a base compound including iron in amount of 10 mass ppm or greater, removal or reduction of iron by using activated carbon, a chelated ion-exchange resin, a chelating agent, or the like; and (ii) carrying out addition of iron to the base compound so that the base compound includes iron in amount between 0.01 to 10 mass ppm.

The amount of iron greater than the above ranges is not preferable, since it ends up with the coloring(s) of the water-absorbent resin. Also, in a case where the amount of iron is set to ND (zero), there is a risk that causes an increase in cost, makes an effect of the operation not worth the cost, and slows down a polymerization speed in Redox polymerization or the like.

The amount of Fe can be measured, for example, by an ICP emission spectrometry method set forth in JIS K1200-6. Regarding an ICP emission spectrometry instrument device, ULTIMA manufactured by Horiba, Ltd. and the like are commercially available.

### (6) Impurity in Acrylic Acid

In consideration of a color stability effect or a residual monomer, an amount of protoanemonin and/or furfural to acrylic acid is controlled so as not to be greater than a predetermined amount. The content amount of protoanemonin and/or furfural to acrylic acid is preferably between 0 to 10 mass ppm, more preferably between 0 to 5 ppm, and further preferably between 0 to 1 mass ppm.

For a similar reason, it is preferable to include a smaller amount(s) of aldehyde content other than furfural and/or maleic acid. An amount(s) of aldehyde content other than furfural and/or maleic acid to acrylic acid is preferably between 0 to 5 mass ppm, more preferably between 0 to 3 mass ppm, further preferably between 0 to 1 ppm, and particularly preferably of 0 mass ppm (below measurable limits). Examples of aldehyde content other than furfural encompass benzaldehyde, acraldehyde, acetaldehyde, and the like.

In order to achieve a reduction in the residual monomer, acrylic acid includes dimer acrylate in amount between 0 to 500 mass ppm, preferably between 0 to 200 mass ppm, and particularly preferably between 0 to 100 mass ppm. Also, in consideration of smell of a resultant water-absorbent resin, both acrylic acid and a water-absorbent resin formed thereof include non-polymeric acetic acid and propionic acid in total amount not greater than 1000 mass ppm, more preferably not greater than 500 mass ppm, and most preferably not greater than 300 mass ppm.

### (7) Method for Producing Acryl Acid

The present invention is not limited to a particular method for producing acrylic acid. For example, the present invention can carry out vapor-phase oxidation with respect to propylene or propane which is used as a material, or can carry out oxidation with respect to a natural product such as glycerin or the like. Such oxidations may be carried out in such a manner as to form acrolein as an intermediate product or may be carried out in such a manner as to isolate acrolein and thereby produce acrylic acid directly.

The water-absorbent resin is used in large quantity in a disposable diaper. In consideration of this, it is preferable to arrange a material for the water-absorbent resin renewable and sustainable. Specifically, it is preferable to prepare the water-absorbent resin and its material from nonfossil raw material. A method for producing such water-absorbent resin and acrylic acid should be a method in which the water-absorbent resin is produced from acrylic acid, which is prepared from glycerin produced from grease.

Examples of the method for producing an acrylic acid-based water-absorbent resin from a preferred nonfossil raw material are disclosed in, for examples, International Publication No. 2006/092273, International Publication No. 2006/136336, International Publication No. 2008/02304, International Publication No. 2007/109128, and the like. Also, examples of the method for preparing acrylic acid from a nonfossil raw material are disclosed in International Publication No. 2006/08024, U.S. Patent Application Publication No.2007/0129570, International Publication No. 2007/119528, International Publication No. 2007/132926, and the like. As the nonfossil raw material, glycerin, lactic acid (also referred to as α hydroxypropionic acid), β hydroxypropionic acid, or the like is used preferably in view of a yield and/or a purity of acrylic acid as well as stable availability of a source for the raw material. Among them, glycerin is particularly used. Lactic acid and/or β hydroxypropionic acid as the raw material for acrylic acid is prepared by performing fermentation of glucose or the like, and glycerin as the raw material for acrylic acid is prepared by carrying out saponification or ester exchange of natural grease. Lactic acid or β hydroxypropionic acid is turned into acrylic acid, when being dehydrated into a salt form or a non-neutralization state. It is preferable to prepare acrylic acid from a natural product, especially grease, since this makes it easier to control an amount of phenol to be included.

That is, in consideration that the raw material is preferably renewable and sustainable and that it is easier to control the amount of phenol to be included, it is preferable to prepare acrylic acid from a raw material (nonfossil raw material) derived from a natural product, and is more preferable to prepare acrylic acid from a material derived from glycerin (natural product). Use of such acrylic acid provides polyacrylic acid-based water-absorbent resin which is sustainable and renewable and derived from natural glycerin.

### (8) Phenol

In the present invention, it is demonstrated that phenol is effective in making the water-absorbent resin more resistible to the early-phase coloring.

Thus, in the present invention, it is preferable to include phenol (rational formula; C₆H₅OH) in the monomer. An amount of phenol in the monomer (solid content) is preferably between 0.01 to 5000 mass ppm, more preferably between 0.01 to 2500 mass ppm, and most preferably between 0.01 to 400 mass ppm. If the amount of phenol in the monomer or acrylic acid to be used is more than 5000 mass ppm, there may be a problem that causes the water-absorbent resin to be changed more in color over time or a problem that gives rise to a phenol odor of the water-absorbent resin. On the other hand, if the amount of phenol is small, especially smaller than 0.01 mass ppm, it is more likely to be impossible to make the water-absorbent resin to be sufficiently resistible to the early-phase coloring (the coloring of the water-absorbent resin which occurs to the water-absorbent resin fresh out of the plant).

Methoxyphenol disclosed in Patent Literatures 4 and 14 and steric barrier phenol disclosed in Patent Literature 10 can be used as a polymerization inhibitor. However, use of an increased amount of methoxyphenol or the like causes the coloring of the water-absorbent resin. In the present invention, in contrast, phenol is used in place of methoxyphenol or steric barrier, thereby preventing the coloring of the water-absorbent resin.

A global standard version of commercially available acrylic acid includes, as a polymerization inhibitor, p-methoxyphenol in an amount of 200 mass ppm. However, p-methoxyphenol makes the water-absorbent resin more vulnerable to the early-phase coloring (the coloring of the water-absorbent resin which occurs to the water-absorbent resin fresh out of the plant). Thus, in view of a stability or a polymerizability of a monomer, a material including p-methoxyphenol has difficulty in giving the water-absorbent resin a higher resistivity to the early-phase coloring. Surprisingly, however, it is found in the present invention that phenol (i.e., non-substituted phenol), addition or inclusion of which in acrylic acid is not known in a conventional art, can make the water-absorbent resin more resistible to the early-phase coloring. The effect of phenol is more noticeable in a case where p-methoxyphenol, especially p-methoxyphenol in a predetermined amount not higher than 200 mass ppm, is used for the polymerization inhibitor. Further, in case of reducing p-methoxyphenol for the prevention of the early-phase coloring, it is preferable to keep an amount of hydroxyacetone small, since this makes it possible to maintain and enhance the stability of the monomer not having been subjected to the polymerization yet. In the present invention in which the control of phenol or hydroxyacetone is carried out unlike in case with Patent Literatures 17 and 18, acrylic acid or the monomer includes the polymerization inhibitor in amount between 10 to 200 mass ppm (with respect to acrylic acid), preferably between 25 to 200 mass ppm, more preferably between 25 to 160 mass ppm, still preferably between 25 to 100 mass ppm, and most preferably between 25 to 70 mass ppm. A preferable example of the polymerization inhibitor includes methoxyphenol, particularly p-methoxyphenol. Use of an increased amount of p-methoxyphenol for the polymerization inhibitor causes a delay in the polymerization or ends up with the coloring of the water-absorbent resin. On the other hand, use of a reduced amount of p-methoxyphenol for the polymerization inhibitor causes a delay in the polymerization as well. In a case where no polymerization inhibitor (especially p-methoxyphenol) is used, the stability of the monomer is lowered even if the amount of hydroxyacetone is controlled so as not to be larger than 300 mass ppm. In this case, furthermore, there may be caused a delay in polymerization reaction, depending on a polymerization condition. Use of the polymerization inhibitor in amount larger than 200 mass ppm may possibly both cause the delay in the polymerization and end up with the coloring (early-phase coloring) of a water-absorbent resin particle. In this case, the water-absorbent resin particle is changed in color into yellow (coloring over time) by a serious degree, especially when it is stored for an extended period of time.

A solubility (8.4 g/100 ml, 20°C) of phenol to water is slightly low. Thus, in the present invention, it is preferable that acrylic acid, which has a high solubility to water, include a predetermined amount of phenol in advance. An increased amount of phenol makes the water-absorbent resin more resistible to the early-phase coloring and, in some cases, gives a rise to an antibiotic property and odor elimination. However, the use of increased amount of phenol has a risk that causes the water-absorbent resin to be changed more in color over time.

Use of phenol prevents a problem that causes the deterioration in the physical property of the water-absorbent resin or the decrease in the stability of the monomer (gelatification or partial gelatification to occur prior to the polymerization causes clogging of a pipe or a tank), and makes it possible to produce the water-absorbent resin white in color, even under conditions highly apt to cause the coloring of the water-absorbent resin. The conditions highly apt to cause the coloring include polymerization of a monomer having a high concentration, polymerization started at high temperature, drying at high temperature, surface cross-linking at high temperature, and the like.

Examples of a method for including a predetermined amount of phenol in acrylic acid include (i) a method in which the predetermined amount of phenol is added to acrylic acid that has subjected to sufficient purification, and (ii) a method in which phenol is generated as a by-product at an intermediate step of a preparation process of acrylic acid, and then, phenol is treated by oxidization (oxygen oxidization) or removed and eliminated so that phenol in acrylic acid is of the predetermined amount. In a method for producing acrylic acid including such preferable phenol, acrylic acid is produced from a raw material derived from a natural product, more preferably from a raw material derived from glycerin (natural product). Use of acrylic acid thus produced provides a polyacrylic acid (salt)-based water-absorbent resin which is sustainable and renewable and derived from natural glycerin.

### (9) Hydroxyacetone

In the present invention, it is found that hydroxyacetone causes a negative effect both in the coloring of the water-absorbent resin occurring over time and in the stability of monomers.

Thus, it is preferable that acrylic acid of the present invention include, as impurity, 0 to 300 mass ppm (in relation to an amount of acrylic acid) of preferably hydroxyacetone (CH₃COCH₂OH; also referred to as acetol). Regarding an upper limit of an amount of hydroxyacetone, 200 mass ppm, 100 mass ppm, 50 mass ppm, 20 mass ppm, 10 mass ppm, 5 mass ppm, 1 mass ppm, and 0.1 mass ppm are preferable in this order. Approximately 0.1 mass ppm of hydroxyacetone can be present in acrylic acid, since preparation of highly-purified acrylic acid gives rise to a problem regarding cost or yield, and since inclusion of a predetermined amount of hydroxyacetone in acrylic acid may give a higher resistivity to the early-phase coloring (especially WB) of the water-absorbent resin, as later discussed in Examples. However, in a case where more than 300 mass ppm of hydroxyacetone is included in a monomer or acrylic acid for use in a process, it becomes easier that the monomer forms a small amount of a polymer as its by-product. The polymer thus formed as the by-product of the monomer causes clogging of a pipe by a small amount of a gel or the like, and makes the water-absorbent resin more vulnerable to the change in color over time (the coloring of the water-absorbent resin which occurs in (i) a storage period between the production of the water-absorbent resin and the use of it and/or (ii) at time of transportation of the water-absorbent resin). Thus, in view of industrially stable production and the physical property of the water-absorbent resin, it is not preferable to include more than 300 mass ppm of hydroxyacetone in the monomer or acrylic acid for use in the operation.

In the present invention, by reducing the amount of hydroxyacetone, it is possible to reduce non-polymeric acetic acid, which is an oxidative product of hydroxyacetone. This in turn makes it possible to reduce acid smell of the water-absorbent resin to be produced. Thus, in the present invention, it is preferable to reduce the amount of hydroxyacetone.

According to the conventional art, partial gelatification is caused (i) in a case where it employs, in consideration of a physical property and/or productivity, an increase in temperature at which polymerization is started, an increase in a concentration of the monomer to be polymerized, and/or an increase in polymerization scale, or (ii) in a case where it employs a decrease in an amount of the polymerization inhibitor in the purpose of preventing the coloring of the water-absorbent resin. Regarding this, it is found in the present invention that hydroxyacetone, as a causal substance, causes a deterioration in the stability of the monomer. In consideration, it is preferable to set the amount of hydroxyacetone in acrylic acid to a range approximately between 0.1 to 10 mass ppm.

That is, by focusing attention on hydroxyacetone, which is a minor component in acrylic acid, the inventors of the present invention found that (i) hydroxyacetone causes the deterioration in the stability of the monomer yet to be polymerized, and is a cause of the change in color of the water-absorbent resin over time, and (ii) since acrylic acid derived from natural glycerin contains a relatively large amount of hydroxyacetone, the water-absorbent resin to be produced from such acrylic acid is likely to be changed in color over time. Based on the findings, the present invention has been made. Considering that acrylic acid prepared from a natural product, particularly glycerin, is likely to contain the relatively large amount of hydroxyacetone, the method of the present invention is suitably used.

### (10) Controlling Method

Phenol (boiling temperature of 182 °C and melting temperature of 43 °C) or hydroxyacetone (boiling temperature of 146 °C and melting temperature of -6 °C) in acrylic acid (boiling temperature of 142 °C and melting temperature of 12 °C) should be controlled by crystallization or distillation, by taking advantage of differences of boiling temperatures, melting temperatures, and solubilities. Alternatively, phenol or hydroxyacetone in acrylic acid should be controlled by adding a predetermined amount of phenol or hydroxyacetone, or carrying out oxidation of phenol or hydroxyacetone. For example, it is preferable to carry out the removal or control of phenol or hydroxyacetone in acrylic acid in accordance with a method disclosed in International Publication No. 2008/053646 (International Publication Date; May 8, 2008), which was not open to the public at the time of the filing of the subject application.

The controlling method disclosed in International Publication No. 2008/053646 (see paragraphs [0015] to [0039] and Example described in paragraphs [0042] to [0054]) is entirely incorporated in the present application. International Publication No. 2008/053646 discloses a method for producing preferred acrylic acid from a natural product. However, in the method is directed to a technique in which phenol or hydroxyacetone in acrylic acid is removed, and International Publication No. 2008/53646 does not disclose, anywhere in the specification including Examples and the detailed description, any technique in which a predetermined amount of phenol or hydroxyacetone is included in acrylic acid and a water-absorbent resin is produced by a particular step. Both Patent Literatures 17 and 18 discloses a technique in which a water-absorbent resin is prepared by carrying out polymerization of acrylic acid derived from glycerin. However, like International Publication No. 2008/053646, Patent Literatures 17 and 18 discloses neither (i) a technique in which a predetermined amount of phenol or hydroxyacetone is included in acrylic acid, and a water-absorbent resin is prepared by a particular step, nor (ii) an arrangement in which a specific polymerization inhibitor (preferably, 25 to 200 mass ppm of p-methoxyphenol) or Fe (preferably, 0.005 to 3 mass ppm (Fe₂O₃ basis) of Fe) is used in combination for control of phenol or hydroxyacetone.

That is, a part of the present invention can incorporate, in addition to the method in (7), a production method of International Publication 2008/053646 as an example of the method for producing acrylic acid. The production method of International Publication No. 2008/053646 is a production method for producing acrylic acid, which includes: a purification step in which phenol and/or 1-hydroxyacetone in an acrolein-containing composition matter is removed; and an oxidation step in which oxidation of the acrolein-containing composition matter thus subjected to the purification step is carried out so that acrylic acid is produced. Preferably, the production method includes, prior to the purification step, a draining step in which acrolein is prepared by carrying out draining of glycerin. In the draining step, it is preferable to carry out the draining of glycerin in a gas phase, In the draining step, it is preferable to carry out the draining of glycerin in a gas phase. In acrylic acid thus produced by the production method, acetic acid can be included in a reduced amount. Thus, acid smell of a water-absorbent resin, which is formed from acrylic acid, can be reduced. Of the water-absorbent resin, a total amount of acetic acid and propionic acid is not larger than 1000 mass ppm, preferably not larger than 500 mass ppm, and particularly preferably not larger than 300 mass ppm.

### (11) Monomer Concentration

Polymerization of the monomer is generally carried out in an aqueous solution. A concentration of a solid content of the monomer in the aqueous solution is generally between 10 to 90 mass %, preferably between 20 to 80 mass %, more preferably between 30 to 70 mass %, and most preferably between 40 to 60 mass %. In case of carrying out the polymerization of the monomer in the aqueous solution, it is optional to add, to the monomer, 0 to 30 mass % (with respect to the monomer) of a high polymer compound, that of a chelating agent, and that of an additive agent. Examples of the high polymer compound encompass an interfacial active agent, polyacrylic acid (salt) or a starch, and polyvinyl alcohol. In view of productivity and drying cost, it is preferable to increase the concentration of the monomer. However, in case of polymerizing the monomer thus having the high concentration, a conventional technique sometimes gives rise to a problem regarding the coloring of the water-absorbent resin and the stability of the monomer. In contrast, the present invention can also solve the problem. Thus, the present invention is suitably usable in the polymerization of the monomer having the high concentration. The high concentration of the monomer is preferably between 30 to 70 mass % and more preferably between 40 and 60 mass %. Use of the monomer whose concentration is not larger than 20 % makes the present invention rather disadvantageous in terms of a polymerization speed and a drying cost. On the other hand, use of the monomer whose concentration is too high, e.g., 90 mass %, tends to give a decreased absorbency (CRC).

### (12) Other Monomer Component

An aqueous solution of an unsaturated monomer can additionally contain (i) a water-soluble resin (such as a starch, polyacrylic acid (salt), polyethylenimine, or the like) or a water-absorbent resin, in an amount between, for example, 0 to 50 mass %, preferably between 0 to 20 mass %, more preferably between 0 to 10 mass %, and most preferably between 0 to 3 mass %, and (ii) a surfactant, an additive agent later described, or the like, in an amount between, for example, 0 to 5 mass % and preferably between 0 to 1 mass %. It is optional to achieve, by this, an enhancement in physical properties of the water-absorbent resin or a particulate water-absorbent agent.

That is, an amount of the chelating agent, hydroxycarboxylic acid, or a reducible inorganic salt in the water-absorbent resin is preferably between 10 to 5000 mass ppm, more preferably between 10 to 1000 mass ppm, further preferably between 50 to 1000 mass ppm, and most preferably between 100 to 1000 mass ppm. Preferably, the chelating agent must be included.

The chelating agent, hydroxycarboxylic acid, and the reducible inorganic salt usable in the present invention are described in (12) through (14) below.

### (13) Chelating Agent (Preferably, Water-Soluble Organic Chelating Agent)

In case of further desiring an enhancement in a color stability (color stability of a particulate water-absorbent agent stored in hot and humid conditions for an extended period of time) and an enhancement in an anti-urine property (gel deterioration prevention), it is preferable to produce the particulate water-absorbent resin of the present invention by use of a chelating agent.

In consideration of an effectiveness, the chelating agent is preferably a water-soluble organic chelating agent, more preferably a non-polymeric compound organic chelating agent including a nitrogen atom or a phosphorus atom, and most preferably an aminopolyhydric carboxylic acid-based chelating agent or an aminopolyhydric phosphoric acid-based chelating agent. In consideration of an effect on polymerization and a physical property to be obtained, the chelating agent is preferably of a non-polymeric-based organic compound having a weight-average molecular weight not larger than 5000, and more preferably of a non-polymeric-based organic compound having a weight-average molecular weight between 100 to 1000.

Among them, a compound having a nitrogen atom or a phosphorus atom is preferable. Further, an aminopolyhydric carboxylic acid (salt) compound having the following range of carboxyl groups in a molecule or an organophosphate acid (salt) compound having a phosphate group is more preferable. The range of carboxyl groups in a molecule is 2 or more, preferably between 3 to 100, more preferably between 3 to 20, and most preferably between 3 to 10. Regarding the organophosphate acid (salt) compound having a phosphate group, a polyorganic phosphate compound or an aminopolyhydric phosphate compound having an amino group is preferable.

Examples of aminopolyhydric carboxylic acid (salt) having 2 or more carboxyl groups encompass aminocarboxylic acid-based metal chelating agents such as iminodiacetic acid, hydroxyethyl iminodiacetic acid, nitrilotriacetic acid, nitrilotripropionic acid, ethylenediaminetetraacetic acid, hydroxy ethylenediaminetriacetic acid, hexamethylenediamine tetraacetic acid, diethylenetriaminepentaacetic acid, triethylenetetraminehexaacetic acid, salts thereof, and the like.

Examples of a polyorganic phosphate compound having 3 or more phosphate groups in a molecule or that of an aminopolyhydric organophosphate compound encompass ethylene diamine-N,N'-di(methylenephosphine acid), ethylenediamine tetra(methylenephosphine acid), polymethylenediamine tetra(methylenephosphine acid), diethylenetriaminepenta (methylenephosphine acid), 1-hydroxyethylidene diphosphonic acid, and salts thereof.

### (14) Hydroxycarboxylic Acid Compound

In order to obtain a color stability effect, it is optional to prepare the monomer or the polymer thereof from hydroxycarboxylic, particularly non-polymer hydroxycarboxylic (salt), disclosed in International Application No. 2007/JP/67348 (International Application Date: August 30, 2007), which was not open to the public at the time of filing of the subject application. Examples of hydroxycarboxylic disclosed therein encompass lactic acid (salt), citric acid (salt), malic acid (salt), and the like.

### (15) Reducing Inorganic Salt

In consideration of a color stability effect, a reducing inorganic salt described in U.S. Patent Application Publication No. 2006/88115 can be used.

### (16) Polymerization Step (Cross-Linking Polymerization Step)

In consideration of performance or an easiness of control of polymerization, the polymerization step is generally carried out by aqueous polymerization or reverse phase suspension polymerization. Particularly, the polymerization step is carried out by (i) the aqueous polymerization in which a conventional technique has a difficulty in carrying out the control of polymerization or preventing the coloring, or further by (ii) continuous aqueous polymerization. Particularly, it is possible to carry out suitable control by continuous polymerization in which by polymerizing one line of an unsaturated monomer solution, a water-absorbent resin is produced in an enormous scale not smaller than 0.5 t/hr, preferably not smaller than 1t/hr, more preferably not smaller than 5t/hr, and particularly preferably not smaller than 10 t/hr. Thus, preferable examples of the continuous polymerization encompass continuous kneader polymerization (e.g., U.S. Patent No. 6987151 and U.S. Patent No. 670141) and continuous belt polymerization (e.g., U.S. Patent No. 4893999, U.S. Patent No. 6241928, and U.S. Patent Application Publication No. 2005/215734).

Preferable example of the continuous polymerization or batch polymerization, particularly the continuous polymerization, encompass polymerization which is carried out at high temperature (temperature of a monomer is not lower than 30 °C, not lower than 35 °C, preferably not lower than 40 °C, and still preferably not lower than 50 °C, and an upper limit of the temperature of the monomer is of a boiling point), and polymerization which is carried out onto a monomer having a high concentration (a concentration of the monomer is not lower than 30 weight %, preferably not lower than 35 weight %, still preferably not lower than 40 weight %, particularly preferably not lower than 45 weight %, and an upper limit of the concentration of the monomer is of a saturated concentration).

Highest maximum during the polymerization is higher than 100 °C, preferably between 103 to 120 °C and more preferably between 105 to 115 °C. If the maximum temperature during the polymerization does not fall in any of the ranges, a water-absorbent resin to be produced may be deteriorated in an absorbency, a water-soluble content, or coloring. Both Patent Literatures 17 and 18 (regarding Patent Literature 18, see Example 3) disclose (i) low-temperature polymerization, such as polymerization which is started at 4 °C, polymerization in which polymerization peak is reached at 100 °C, or the like, and (ii) low-temperature drying, such as drying which is carried out at 150 °C or the like. In contrast, the present t invention is suitably applicable to high-temperature polymerization and high-temperature drying.

With the present invention in which a predetermined amount of phenol or hydroxyacetone can be prepared even in the polymerization carried out at high temperature or the polymerization carried out onto the monomer having the high concentration, it is possible to obtain a water-absorbent resin white in color. Therefore, the present invention brings about a more noticeable effect when applied in such polymerizations. Preferable examples of the polymerization started at high temperature are disclosed in U.S. Patent No. 6906159 and U.S. Patent No. 7091253, and the like. With the method of the present invention in which an excellent stability of a monomer not subjected to any polymerization can be obtained, it is easier to produce a water-absorbent resin in a plant scale.

These polymerizations can be carried out at air atmosphere. However, in view of the prevention of the coloring, it is preferable to carry out the polymerization at inert gas atmosphere (e.g., oxygen concentration not higher than 1 %), and to polymerize a monomer component after sufficiently replacing, by an inert gas, oxygen contained in a monomer or oxygen dissolved in a solution containing the monomer (e.g., after reducing oxygen lower than 1 ppm). As seen in the above, even in case of carrying out degassing, it is possible with present invention to secure an excellent monomer stability and to prevent gelatification from occurring prior to polymerization, and thereby to provide a water-absorbent resin excellent in physical properties and white in color.

### (17) Polymerization Initiator

A polymerization initiator for use in the present invention is selected as needed in accordance with a form of the polymerization. Examples of the polymerization initiator encompass a photodegradable polymerization initiator, a pyrolytic polymerization initiator, and a redox-type polymerization initiator. An amount of the polymerization initiator to the monomer is between 0.0001 to 1 mol % and preferably between 0.001 to 0.5 mol %.

Use of an increased amount of the polymerization initiator may end up with the coloring of the water-absorbent resin, whereas use of a reduced amount of the polymerization initiator causes an increase in a residual monomer. There is a case that use of a conventional color preventing agent gives a negative effect to polymerization. In contrast, the method of the present invention can make the water-absorbent resin more resistible to the coloring, without giving no negative effect to the polymerization (conventional time, physical properties, or the like). Therefore, the method of the present invention is preferable.

Examples of the photodegradable polymerization initiator encompass a benzoin derivative, a benzyl derivative, an acetophenone derivative, a benzophenone derivative, and an azo compound. Examples of the pyrolytic polymerization initiator encompass persulfate (sodium persulfate, potassium peroxodisulfate, or ammonium persulfate), a peroxide (hydrogen peroxide, t-butylperoxide, or methylethylketoneperoxide), and an azo compound (2,2'-azabis (2-amidino-propane) dihydrochloride, 2.2'- azobis [2-imidazoline-2-yl) propane] dihydrochloride, or the like).

An example of the redox-type polymerization initiator encompasses use of a reducing compound (such as L-ascorbic acid or sodium bisulfite) in combination with persulfate or peroxide. A preferable example of the redox-type polymerization initiator encompasses use of the photolytic polymerization initiator in combination with the pyrolytic polymerization initiator.

### (18) Gel Grain Refining Step

A cross-linked polymer in an aqueous gel phase, which is obtained by the polymerization step, can be dried directly. However, in the present invention, it is crushed into particles by a crusher (kneader, meat chopper, or the like) during the polymerization or after the polymerization, as required.

In view of physical properties, the aqueous gel to be crushed is kept or heated preferably between 40 to 95 °C and more preferably between 50 to 80°C. A resin solid content of the aqueous gel is not particularly limited. However, in view of the physical properties, it is preferably between 10 to 70 mass %, more preferably between 15 to 65 mass %, and most preferably between 30 to 55 mass %. It is optional to add water, polyhydric alcohol, a mixture solution of water and polyhydric alcohol, a solution prepared by dissolving polyvalent metal in water, vapors thereof, or the like.

### (19) Drying Step

In order to reduce the residual monomer in amount, to prevent the gel deterioration (anti-urine property), and to prevent the yellowing as objects of the present invention, it is preferable to employ an arrangement in which a time span between the polymerization and a drying step via the gel crushing step carried out as required is short. That is, the drying step, which is carried out with respect to the cross-linked polymer in the aqueous gel phase after the polymerization, is started (the cross-linked polymer in the aqueous phase is fed into a drier) preferably within an hour, more preferably within 0.5 hour, and still more preferably within 0.1 hour. Further, in order to reduce the residual monomer or to allow the water-absorbent resin to be less colored, a temperature of the cross-linked polymer during the time span between the polymerization and the drying step is preferably between 50 to 80 °C and more preferably between 60 to 70 °C.

The drying step provides a dry product having a solid resin content in amount, which is calculated from a drying loss of the dry product, preferably not less than 80 mass %, more preferably between 85 to 99 mass %, further preferably between 90 to 98 mass %, particularly preferably between 92 to 97 mass %. In the drying step, drying temperature is not particularly limited. However, the drying temperature is preferably between 100 to 300 °C and more preferably between 150 to 250 °C. In order to secure the high physical properties and whiteness in color, it is most preferable to arrange the drying step so that the drying is carried out at drying temperature between 165 to 230 °C for drying time within 50 minutes. If the drying temperature or the drying time does not fall in the range above, there is a risk that causes a deterioration in the absorbency (CRC), an increase in the extractables, and a decrease in whiteness index.

For the drying method, it is possible to use a variety of methods including (i) drying by heating, (ii) hot-air drying, (iii) reduced-pressure drying, (iv) infrared ray drying, (v) microwave drying, (vi) drying with a drum dryer, (vii) draining by azeotropy with hydrophobic organic solvent, (viii) high humid draining in which hot water vapor is used, and the like. Particularly, it is preferable to use hot-air drying in which a gas, whose dew point is preferably between 40 to 100 °C and more preferably between 50 to 90 °C, is used. While a conventional technique is easier to pose a problem regarding the early-phase coloring, the present invention in which phenol is used realizes the high physical properties and less coloring by such high-temperature drying.

### (20) Crushing Step or Sieving Step (Particle Size and Adjustment thereof After the Drying)

It is optional to carry out adjustment of a particle size in accordance with necessity, after the drying step in which the cross-linking polymer in aqueous gel phase is dried. In the adjustment of a particle size, it is preferable to adjust the particle size to a specific value in order to improve a physical property in surface cross-linking later described. The adjustment of the particle size can be carried out by polymerization (particularly the reverse phase suspension polymerization), crushing, sieving, granulation, removal of a fine powder, or the like, as needed. In the description discussed hereinafter, the particle size is defined by a standard sieve (JIS 28801-1 (2000)).

The adjustment of the particle size is carried out so that a mass average particle diameter (D50) to be obtained prior to the surface cross-linking is between 200 to 600 µm, preferably between 200 to 550 µm, more preferably between 250 to 500 µm, and particularly preferably between 350 to 450 µm. It is preferable that a resultant of the adjustment contain a reduced amount of particles whose particle diameter is smaller than 150 µm. An amount of the particles with such range of the particle diameter is generally between 0 to 5 mass %, preferably between 0 to 3 mass %, and most preferably between 0 to 1 mass %. Also, it is preferable that the resultant of the adjustment contain a reduced amount of particles whose particle diameter is not less than 850 µm. An amount of the particles with such range of the particle diameter is generally between 0 to 5 mass %, preferably between 0 to 3 mass %, and most preferably between 0 to 1 mass %. A logarithmic standard deviation (σζ) of particle size distribution is preferably between 0.20 to 0.40, more preferably between 0.27 to 0.37, and further preferably between 0.25 to 0.35. Methods for above measurement by use of the standard sieve is disclosed, for example, in International Publication No. 2004/69915 and EDANA-ERT 420.2-02.

Generally, coloring of the particles at the time of the measurements becomes more noticeable, if the particle size distribution is narrow due to the adjustment of the particle size, i.e., if upper and lower limits of the particle size are controlled by the adjustment of the particle size. However, the present invention does not pose such problem, and is therefore preferable. The present invention preferably includes, after the drying step, a sieving step in which the adjustment of the particle size is carried out so that the resultant of the adjustment of the particle size contains a particle composition, whose particle diameter is between 150 to 850 µm according to the sieving with the standard sieve, in amount not less than 95 mass % and preferably not less than 98 mass % (an upper limit of 100 mass %). Likely, the resultant of the adjustment of the particle size contains a particle composition, whose particle size is between 300 to 600 µm, in amount preferably not less than 50 mass %, still preferably not less than 60 mass, and particularly preferably not less than 70 mass %. The standard sieve for use in the measurements is JIS Z8801-1 (2000) or a corresponding sieve.

### (21) Surface Cross-Linking Step

In the present invention, it is preferable to further include a surface cross-linking step after the drying step. With the method of the present invention for producing the water-absorbent resin, it is possible to obtain a water-absorbent resin which is more resistible to further coloring due to the surface cross-linking and is thereby whiter in color. The method of the present invention is suitably applicable in a production operation of a water-absorbent resin which employs surface cross-linking, especially in a production operation of a water-absorbent resin which employs cross-linking carried out at high temperature.

Examples of a surface cross-linking agent useable in the present invention encompass a variety of organic or inorganic cross-linking agents. Among them, an organic surface cross-linking agent can be preferably used. The surface cross-linking agent can be preferably of a polyhydric alcohol compound, an epoxy compound, a polyhydric amine compound, a condensate of a polyhydric amine compound and a haloepoxy compound, an oxazolidine compound, a (mono, di, or poly)oxazolidine compound, or an alkylene carbonate compound. Particularly, the surface-cross linking agent can be a dehydrated ester reactive cross-linking agent which is composed of a polyhydric alcohol compound, an alkylene carbonate compound, or an oxazolidine compound and which has to be reacted at high temperature.

More concrete examples of the surface cross-linking agent encompass compounds disclosed in U.S. Patent No. 6228930, U.S. Patent No. 6071976, U.S. Patent No. 6254990, and the like. Such compounds are, for example, (i) a polyhydric alcohol compound such as (mono, di, tri, or tetra)propylene glycol or propylene glycol, 1,3-propanediol, glycerin, 1,4-butandiol, 1,3-butandiol, 1,5- pentandiol, 1,6-hexanediol, sorbitol, or the like, (ii) an epoxy compound such as ethyleneglycol diglycidyl ether, glycidol, or the like, (iii) an alkylene carbonate compound such as ethylene carbonate or the like, (iv) an oxetane compound, (v) a cyclic urea compound such as 2-imidazolidinone, and the like.

An amount of the surface cross-linking agent to be added to 100 weight parts of the water-absorbent resin is determined as appropriate in a range approximately between 0.001 to 10 mass parts or in a range approximately between 0.01 to 5 mass parts. Water can be used preferably in accordance with the surface cross-linking agent. An amount of water to 100 mass parts of the water-absorbent resin is between 0.5 to 20 mass parts, more preferably between 0.5 to 10 mass parts.

A hydrophilic organic solvent can be used concurrently with the above, and an amount of the hydrophilic organic solvent to 100 weight parts of a water-absorbent resin particle is between 0 to 10 weight parts, preferably between 0 to 5 weight parts. In adding a cross-linking agent solution to the water-absorbent resin particle, it is optional to allow coexistence of a water-insoluble fine particle powder and a surfactant in a total amount that does not prevent the effects brought about by the present invention, e.g., a total amount between 0 to 10 weight parts or smaller, preferably a range between 0 to 5 weight parts, more preferably a range between 0 to 1 weight part. The surfactant to be used and an amount thereof are disclosed in International Publication No. 2005/075070 (International Application No. 2005/JP/1689 (International Application Date: February 4, 2005)) and corresponding U.S. Patent Application Publication No. 2005/209352.

The water-absorbent resin to which the surface cross-linking agent has been mixed is preferably heated, and, if necessarily, cooled subsequently. A heating temperature is between 70 to 300 °C, preferably between 120 to 250 °C, and more preferably between 150 to 250 °C. A heating time is preferably between 1 minute to 2 hours. Heating can be carried out by use of a drier or a heating oven. With the present invention, it is possible to produce a water-absorbent resin highly white in color, even in case of carrying out high-temperature heating or drying by air (hot air)

In case of using the present invention particularly in a sanitary material (especially a disposable diaper), it is necessary to improve an absorbency against pressure (AAP) of the water-absorbent resin to the following range, preferably 20 g/g or higher, by using the surface cross-linking agent.

### (Ion-Binding Surface Cross-Linking Agent)

It is optional to use an inorganic surface cross-linking agent other than the organic surface cross-linking agent so as to improve a permeability potential or the like of the water-absorbent resin. A usable example of the inorganic surface cross-linking agent encompasses a salt (organic salt or inorganic salt) or a hydroxide of bivalent metal or polyvalent metal, preferably trivalent or tetravalent metal. Usable examples of the polyvalent metal encompass aluminum, zirconium, aluminum lactate, aluminum sulfate, and the like. Such inorganic surface cross-linking agents are used concurrently with the inorganic surface-cross linking agent or used separately from the inorganic surface cross-linking agent. Surface cross-linking by polyvalent metal is disclosed in International Publication No. 2007/121037, International Publication No. 2008/09843, International Publication No. 2008/09842, U.S. Patent No. 7157141, U.S. Patent No. 6605647, U.S. Patent No. 6620889, U.S. Patent Application Publication No. 2005/0288182, U.S. Patent Application Publication No. 2005/0070671, U.S. Patent Application Publication No. 2007/0106013, and U.S. Patent Application Publication No. 2006/073969.

It is optional to use a polyamine polymer concurrently with the organic surface cross-linking agent or separately from it so as to improve a permeability potential of the water-absorbent resin. Particularly, the polyamine polymer to be used is a polyamine polymer having an average molecular weight not less than 3000 or that having an average molecular weight between 5000 to 1000000. Usable examples of such polyamine polymers are disclosed, for example, in U.S. Patent No. 7098284, U.S. Patent No. 6849665, International Publication No. 2006/082188, International Publication No. 2006/082189, International Publication No. 2006/082197, International Publication No. 2006/111403, International Publication No. 2006/111404, and the like.

### (Surface Cross-Linking Agent Prepared from Nonfossil Material)

In the present invention, in case of producing acrylic acid from a nonfossil material, it is preferable in view of sustainability or renewability to use a surface cross-linking agent which is derived from a nonfossil material. The nonfossil material can be polyvalent alcohol such as glycerin or the like, or lactic acid or a salt (a monovalent salt, particularly a polyvalent metal salt or an aluminum salt) thereof. In consideration of a physical property in particular, the surface cross-linking agent is prepared from 1,2-(or 1,3-)-propanediol or lactic acid, and such propanediol or lactic acid is prepared from glycerin or cellulose by carrying out chemical oxidation, reduction, or fermentation (biological oxidation).

For example, glycerin to be used can be of a natural product, a synthetic, or a semi-synthetic. However, in view of sustainability of a material or CO₂ emission, it is preferable to use glycerin derived from a natural product, e.g., saponified grease. Due to a restriction imposed on the CO₂ emission, biodiesel is widely used, and use of glycerin, which is a byproduct of biodiesel, is a preferable example. A method for preparing glycerin from grease is not particularly limited. Examples of the method are disclosed, for example, in U.S. Patent Application Publication No. 2007/0167642, International Publication No. 2007/029851, International Publication No. 2006/088254, and the like.

In the present invention, a water-absorbent resin is produced by using a reduced product of glycerin, preferably propanediol prepared by reducing natural glycerin. Considering that the water-absorbent resin is consumed and disposed in a large quantity, such cross-linking agent is preferable in view of environmental load imposed by the water-absorbent resin or sustainability thereof. Furthermore, this cross-linking agent is advantageous in terms of cost and safety and also causes an improvement in physical properties later described (e.g., absorbency against pressure), as compared to another cross-linking agent prepared by means of a conventional complicating organic synthesis.

The method of the present invention for preparing propanediol is not particularly limited. Methods for preparing 1,3-propanediol from glycerin are disclosed in Japanese Patent Application Publication, Tokukai, No. 2005-102533, U.S. Patent Application Publication No. 2007/0148749, and the like. Methods for preparing 1,2-propanediol from glycerin are disclosed in U.S. Patent No. 5276181, U.S. Patent Application Publication No. 2005/0244312, Japanese Patent Application Publication, Tokukai, No. 2007-283175, and the like.

### (22) Other Steps

It is optional to include, in accordance with necessity, other steps than the steps discussed above, which include an evaporating-monomer recycling step, a granulation step, a fine-powder removal step, a fine-powder recycling step, and the like. Furthermore, in order to realize the obtainment of a resisting effect to the coloring over time, the prevention of the gel deterioration, or the like, it is also optional to use an additive agent (later described) in the monomer or the polymer thereof.

### (23) Water-Absorbent Resin and Property thereof

The water-absorbent resin of the present invention is an acrylic resin of which acrylic acid is derived from natural glycerin. The present invention provides the water-absorbent resin which shows Y1 ≤ 30 after being subjected to a color acceleration test. Also, the water-absorbent resin contains phenol, and an amount of phenol therein falls in any of the ranges discussed earlier. This makes the water-absorbent resin of the present invention advantageous in terms of the early-phase coloring. Further, in consideration of the resistivity to the coloring over time, the water-absorbent resin of the present invention also contains an additive agent selected from the group consisting of a chelating agent, a reducing inorganic salt, and hydroxycarboxylic acid. Furthermore, in consideration of an antibiotic property, the water-absorbent resin of the present invention also contains acetic acid and propionic acid. Preferable amounts of acetic acid and propionic acid contained in the water-absorbent resin of the present invention fall in the respective ranges discussed earlier. A ratio of acrylic acid derived from a natural product to an entire material is not less than 50 weight %, preferably of 70 weight %, and particularly preferably of 90 mass %. Thus, the water-absorbent resin of the present invention, of which monomer consumable materials are sustainable and renewable, is preferable in terms of sustainability and renewability, and is whiter in color than any other conventional water-absorbent resin.

### (24) Physical Property of Water-Absorbent Resin

In case of intending to use the water-absorbent resin of the present invention in a hygienic good, particularly a disposable diaper, it is preferable to carry out, by means of the polymerization and by use of the cross-linking agent, controlling of at least one of (a) through (e), preferably two or more of AAP and (a) through (e), and more preferably three or more of AAP and (a) through (e). If the following are not met, the water-absorbent resin of the present invention for use in a high-consistency diaper (later described) may not show sufficient performance.

### (a) Early-Phase Coloring

The water-absorbent resin of the present invention is advantageous in terms of the early-phase coloring. For example, in a Hunter Lab surface color system, the water-absorbent resin has (i) an L value (lightness) preferably not less than 85, more preferably not less than 87, and still preferably not less than 89, (ii) a b value between -5 to 10, preferably between -5 to 5, and still preferably between -4 to 4, and (iii) an a value between -2 to 2, at least between -1 to 1, preferably between -0.5 to 1, and most preferably between 0 to 1. Also, the water-absorbent resin of the present invention has a YI not greater than 10, preferably not greater than 8, and particularly preferably not greater than 6, and a WB not less than 70, preferably not less than 75, and particularly not less than 77. Further, the water-absorbent resin of the present invention is also advantageous in terms of the coloring over time. As such, it secures a sufficient white index even when being left in a hot and humid condition, which is employed in a color acceleration test (model) for examining a color stability of the water-absorbent resin with respect to storage over an extended period of time. It is preferable to use one or more of the chelating agent, the reducing agent, and hydroxycarboxylic acid in addition to the use of phenol or hydroxyacetone, in order to achieve a further improvement in the resistibility to the change in color over time or an improvement in a gel stability (also referred to as an anti-urine property).

### (b) Absorbency Against Pressure (AAP)

In some cases, in order to prevent a leakage from a diaper, the present invention carries out control of absorbency (AAP) by the polymerization step in such a manner that AAP with respect to a 0.9 mass % sodium chloride aqueous solution at 1.9 kPa or 4.8 kPa (approximately 50 g/cm²), both of which are set forth in ERT 442.2-02, is preferably not less than 20 (g/g), more preferably not less than 22 (g/g), and still preferably not less than 24 (g/g). Higher AAP is more preferred, but an increase in AAP trades off against other properties in some cases. In consideration of a balance between AAP and other properties or producing cost, an upper limit of AAP is set at 40 g/g or 35 g/g. Further, regarding AAP with respect to the 0.9 mass % sodium chloride aqueous solution at 4.8 kPa, even an upper limit of 30 g/g is satisfactory. It is possible to obtain AAP within any of the upper limits by, for example, carrying out surface cross-linking of the water-absorbent resin (e.g., having an amount of the particle composition, whose particle diameter is between 150 µm to 850 µm, not less than 95 mass %) having particle sizes in any of the ranges described earlier, in such a manner that the surface cross-linked water absorbent resin has CRC later described. That is, such AAP can be obtained by carrying out the surface cross-linking in such a manner that CRC obtained after the surface cross-linking is lower, preferably by a degree not less than 1 g/g or a degree between 2 to 15 g/g, than CRC obtained prior to the surface cross-linking.

### (c) Permeability Potential (SFC)

In some cases, the present invention carries out control of SFC by the polymerization step so as to prevent a leakage from a diaper, where SFC is a flow conductivity with respect to 0.69 mass % of sodium chloride aqueous solution. The present invention carries out the control of SFC in such a manner that SFC is not less than 1 (cm³·s·10⁻⁷/g), preferably not less than 10 (cm³·s·10⁻⁷/g), more preferably not less than 50 (cm³·s·10⁻⁷/g), still preferably not less than 70 (cm³·s·10⁻⁷/g), and particularly preferably not less than 100 (cm³·s·10⁻⁷/g). Likely to AAP, higher SFC is more preferred, but an increase in SFC trades off against other properties in some cases. Thus, an upper limit of SFC is set approximately 1000 (cm³·s·10⁻⁷/g), in consideration with a balance between SFC and other properties or producing cost. It is possible to obtain AAP within the upper limit by, for example, carrying out surface cross-linking of the water-absorbent resin (e.g., having an amount of the particle composition, whose particle diameter is between 150 to 850 µm, not less than 95 mass %) having particle sizes in any of the ranges described earlier, in such a manner the surface cross-linked water-absorbent resin has CRC in a numeric range later described. That is, such SFC can be obtained by carrying out the surface cross-linking in such a manner that CRC obtained after the surface cross-linking is lower, preferably by 1 g/g or more and still preferably by 2 to 15 g/g, than CRC obtained prior to the surface cross-linking.

### (d) Absorbency Without Pressure (CRC)

An absorbency without pressure (CRC), which is set forth in ERT 441.2-02, is controlled so as preferably not to be less than 10 (g/g), more preferably not to be less than 20 (g/g), still preferably not to be less than 25 (g/g), and most preferably not to be less than 30 (g/g). Higher CRC is more preferred, and an upper limit on CRC is not particularly limited. However, in consideration of a balance between CRC and other physical properties, CRC is preferably not higher than 50 (g/g), more preferably not higher than 45 (g/g), and most preferably not higher than 40 (g/g). Low CRC is disadvantageous in terms of an absorption amount (particularly in absorption of urine in a diaper), whereas an increase in CRC trades of against other properties including AAP and SFC in some cases. Thus, in consideration of a balance between CRC and other properties or producing cost, an upper limit of CRC is set at 45 g/g. For CRC, even an upper limit of 40 g/g is satisfactory. Thus, a preferable range of CRC is between 20 to 40 g/g, and a more preferable range of CRC is between 25 to 40 g/g. The present invention carries out the control of CRC in any of the ranges particularly by the surface cross-linking (a surface cross-linking agent and an amount thereof, as well as reaction temperature and reaction time), in addition to carrying out such control of CRC by a concentration, an amount of a cross-linking agent, and drying temperature in the polymerization.

### (e) Amount of Water-Extractables (Extractables)

An amount of water-extractables is preferably higher than 0 mass % but not higher than 35 mass %, more preferably not higher than 25 mass %, still preferably not higher than 15 mass %, and most preferably not higher than 10 mass %.

### (f) Residual Monomer

In a case where any of the polymerization discussed so far is used as achieving means, an amount of the residual monomer is preferably between 0 to 400 mass ppm, more preferably between 0 to 300 mass ppm, and most preferably between 0 to 200 mass ppm.

### (25) Other Additive Agents

In accordance with a purpose, the water-absorbent resin can contain a substance in a range between 0 to 3 mass % and preferably between 0 to 1 mass %. Examples of the substance encompass an oxidant, an antioxidant, water, a polyvalent metal compound, a water-insoluble inorganic or organic compound such as silica, a metal soap, or the like, a deodorant agent, an antibacterial agent, polymer polyamine, a pulp, a thermoplastic fiber, and the like.

### (26) Purpose of Use

A purpose of use of the water-absorbent resin of the present invention is not particularly limited. However, it is preferable to arrange the water-absorbent resin of the present invention usable in an absorbing article such as a disposable diaper, a sanitary napkin, an incontinence pad, or the like. The water-absorbent resin of the present invention exhibits a particularly excellent performance, in case of (i) being used in a high-consistency diaper (one diaper in which a large amount of a water-absorbent resin is used) that conventionally suffers an odor, coloring, and the like due to a material for a particulate absorbent, and particularly in case of (ii) being used in a top layer portion of an absorbent of the absorbing article.

It is possible to obtain the effect of the present invention in case of including, in the absorbent of the absorption article, the water-absorbent resin of the present invention in an amount (core concentration) between 30 to 100 mass %, preferably between 40 to 100 mass %, more preferably between 50 to 100 mass %, still preferably between 60 to 100 mass %, particularly preferably between 70 to 100 mass %, and most preferably between 75 to 95 mass %. In case of using the water-absorbent resin of the present invention in any of the ranges, particularly in case of using it for the top layer portion of the absorbent, it is possible to give the absorbent a high permeability potential (permeability potential against pressure). The absorbent thus given the high permeability potential has an excellent diffusion property with respect to absorbed liquid such as urine or the like. For this, it is possible to provide an absorbing article such as a disposable diaper or the like, which has an increased absorption capacity due to an efficient liquid distribution and whose absorbent is kept white in color and thereby gives cleanness impression.

It is preferable that the absorbent be formed by compression so as to have a density between 0.06 to 0.50 g/cc and a basis weight between 0.01 to 0.20 g/cm². The absorbent has a thickness not thicker than 30 mm, preferably not thicker than 20 mm, and more preferably not thicker than 10 mm. By this, it is possible to provide the absorbent suitable for a disposable diaper reduced in thickness.

### [Examples]

### (Evaluation of Physical Property)

### (a) Early-Phase Coloring

The early-phase coloring of the water-absorbent resin was measured by use of a spectroscopic colorimeter SZ-Σ80 COLOR MEASURING SYSTEM (NIPPON DENSHOKU INDUSTRIES CO., LTD) which was set to reflection measurement. Instruments used in the measurement included: a powder/paste container, which was an accessory for the spectroscopic colorimeter SZ-Σ80 COLOR MEASURING SYSTEM and had an inner diameter of 30 mm and a height of 12 mm; a powder/paste white plate No. 2 having a rounded shape, which served as a standard; and a 30 Φ projector pipe. The powder/paste container was filled with approximately 5 g of the water-absorbent resin.

### (b) Coloring Over Time

A water-absorbent resin was left under hot and humid conditions, and then coloring of the water-absorbent resin was measured by the method described in (1).

### (c) Other Physical Properties

An absorbency without pressure (CRC) with respect to 0.9 % normal saline solution, a pH extractables, residual acrylic acid, a permeability potential (SFC) were measured in accordance with EDANA ERT or U.S. Patent Application Publication No. 2006/204755.

### [Production Example 1]

Acrylic acid, which included 60 mass ppm of p-methoxyphenol as a polymerization inhibitor but did not include phenol or hydroxyacetone, was prepared by carrying out distillation and crystallization. The preparation of acrylic acid was carried out so that an amount of a dimer was 100 mass ppm or smaller, an amount of furfural and that of protoanemonin were 1 mass ppm or smaller, and a total amount of acetic acid and propionic acid was 500 mass ppm (which included 400 mass ppm of acetic acid).

An NaOH aqueous solution was prepared from caustic soda including 0.2 mass ppm of iron, and then added with acrylic acid under a coolant state (liquid temperature of 35 °C). By this, 75 mol % neutralization of the NaOH aqueous solution was carried out in a separate step. An amount of iron in acrylic acid and that in water were lower than measurable limits, and a calculated value of an Fe amount in a monomer was approximately 0.07 mass ppm.

### [Example A]

In Production Example 1, an acrylic acid sodium aqueous solution (1) having a neutralization rate of 75 mol % and a concentration of 35 weight % was obtained. In Example A, a monomer (1) was prepared by dissolving, as an internal cross-linking agent, 0.05 mol % (with respect to a monomer) of polyethyleneglycol diacrylate in the acrylic acid sodium aqueous solution (1). 350 g of the monomer (1) was fed in a cylindrical container having a capacity of 1L, and deaerated for 20 minutes by carrying out bubbling of nitrogen at 2L/minute. Then, an aqueous solution of 0.12 g/mol (with respect to the monomer) of sodium persulfate and an aqueous solution of 0.005 g/mol (with respect to the monomer) of L-ascarbic acid were added to the monomer (1) while a reaction solution was being stirred by a stirrer, so that polymerization was started. After the start of the polymerization, the stirring was halted so that polymerization in stationary aqueous solution was carried out. Approximately 15 minutes (polymerization peak time) after a start of the polymerization, a temperature of the monomer (1) reached a peak polymerization temperature of 108 °C. Then, the polymerization was continued for another 30 minutes. After this, the reaction solution was collected from the cylindrical container, and a cross-linked polymer (1) in aqueous gel phase was obtained.

The cross-linked polymer (1) in aqueous phase was fragmented by a meat chopper (pore of 8 mm) at 45 °C, and dried for 20 minutes by a hot air drier being set to 170 °C. Subsequently, a resultant dry polymer (solid content of approximately 95 %) was crushed by a roll mill, and classified in a range between 600 to 300 µm by a JIS standard sieve. By this, a comparative water-absorbent resin (1) as a polyacrylic acid-based water-absorbent resin (neutralization rate of 75 %) was produced.

### [Example 1]

In Production Example 1, 75 mol % neutralization of an NaOH aqueous solution was carried out by using, as acrylic acid which would be contained in a monomer to be obtained in a subsequent step, acrylic acid in which 24 mass ppm of phenol had been dissolved in advance. This prepared a sodium acrylate solution having a neutralization rate of 75 %, and by use of it, a monomer was prepared. The monomer included 20 mass ppm (with respect to the monomer) of phenol. After the preparation of the monomer, polymerization of the monomer was carried out. Both a polymerization peak time and a peak temperature in the polymerization were substantially similar to those in Example A. By the polymerization, a cross-linked polymer in aqueous gel phase was prepared. Then, the cross-linked polymer in aqueous gel phase was dried and crushed in a same way as Example A, so that a water-absorbent resin (1) was produced.

### [Example 2]

In Example 2, 123 mass ppm of phenol had been dissolved in advance in acrylic acid. Acrylic acid thus including 123 mass ppm of phenol was used in place of acrylic acid of Example A, and thus would be included in the monomer to be prepared in a subsequent step. After this, an operation similar to that in Example A was carried out, so that the monomer was prepared. The monomer included 100 mass pm (with respect to the monomer) of phenol. After the preparation of the monomer, polymerization of the monomer was carried out. Both a polymerization peak time and a peak temperature in the polymerization were substantially similar to those in Example A. By the polymerization, a cross-linked polymer in aqueous gel phase was prepared. Then, the cross-linked polymer in aqueous gel phase was dried and crushed in a similar way as Example A, so that a water-absorbent resin (2) was produced.

### [Example 3]

In Example 3, 246 mass ppm of phenol had been dissolved in advance in acrylic acid. Acrylic acid thus including 246 mass ppm of phenol was used in place of acrylic acid of Example A, and thus would be included in a monomer to be prepared in a subsequent step. After this, an operation similar to that in Example A was carried out, so that the monomer was prepared. The monomer included 200 mass ppm (with respect to the monomer) of phenol. After the preparation of the monomer, polymerization of the monomer was carried out. Both a polymerization peak time and a peak temperature in the polymerization were substantially similar to those in Example A. By the polymerization, a cross-lined polymer in aqueous gel phase was obtained. The cross-linked polymer in aqueous gel phase was dried and crushed in a similar way as Example A, so that a water-absorbent resin (2) was produced.

### (Conclusion)

Results of Examples 1 through 3 and Example A are shown in Table 1.

As shown in Table 1, owing to the presence of phenol in the polymerization, reductions in YI (Yellow Index) and a b value as well as an increase in WB (White Balance) of a water-absorbent resin were obtained, regardless of factors such as the polymerizations, physical properties, and stabilities of the monomers. YI of the water-absorbent resin was reduced from 7.1 down to 5.2, WB of the water-absorbent resin was increased from 75.1 up to 78.2, and the b value of the water-absorbent resin was reduced from 3.4 down to 2.7. From this, it was found that use of acrylic acid including phenol, i.e., performing of the polymerization of the monomer in the presence of phenol, made the water-absorbent resin more resistible to the "early-phase coloring (i.e., an increase in a white index was obtained)".

Gelatification of the monomer is more serious, in a case where polymerization is carried out at higher temperature (e.g., starting temperature of 35 °C or higher), where polymerization is carried out onto a monomer having a high concentration (e.g., 40 % or higher), and/or where polymerization is carried out at a larger scale (e.g., 0.5 Mt/hr or higher). In consideration of this, the present invention can be used more suitably. Also, although acrylic acid used in Examples included a total amount of 500 ppm of acetic acid and propionic acid, the polyacrylic acid-based water-absorbent resin thus produced did not smell acid. However, if the total amount of acetic acid and propionic acid in acrylic acid is more than 1000 ppm, a water-absorbent resin to be produced may smell acid.

**[Table 1]**

| Results of Examples 1 through 3 ··· "Effect of Phenol" | | | | | |
|---|---|---|---|---|---|
| | | Example A | Example 1 | Example 2 | Example 3 |
| Additive agent | | None | Phenol | ← | ← |
| Amount (of additive agent) in relation to monomer | ppm | 0 | 20 | 100 | 2000 |
| Peak temp | °C | 108 | 110.3 | 108.5 | 106.8 |
| Color | | | | | |
| Monomer stability | | Good | ← | ← | ← |
| Early-phase coloring | L | 89.1 | 89.6 | 89.8 | 90.3 |
| (immediately after production) | a | 0.0 | 0.1 | 0.1 | -0.4 |
| | b | 3.4 | 3.1 | 2.9 | 2.7 |
| | YI | 7.1 | 6.6 | 6.0 | 5.2 |
| | WB | 75.1 | 76.0 | 77.2 | 78.2 |

| | | | | | |
|---|---|---|---|---|---|
| Note: Polymerization and Physical Property in Comparative Example 1 and Examples 1 through 5 1. within the margin of error, absorbency was approximately 30 g/g, extractables were approximately 17 %, residual monomer was approximately 290 ppm, average particle diameter was 440 mm 2. within the margin of error, peak temperature in polymerization was approximately 109 °C, peak time in polymerization was approximately 15 minutes 3. Hydroxyacetone was ND (not more than 1 ppm) 4. Amount (ppm) of the additive agent was measured in relation to a monomer (75 mol % neutralized sodium acrylate, molar weight of 88.5) | | | | | |

### [Example 4]

Example 4 was different from Example A in that acrylic acid in which 24 mass ppm of hydroxyacetone had been dissolved in advance was used in place of acrylic acid of Example A, and thus included in a monomer prepared in a subsequent step. Thereafter, polymerization similar to that in Example A was carried out. Both a polymerization peak time and a peak temperature in the polymerization were substantially similar to those in Example A. By the polymerization, a cross-linked polymer in aqueous gel phase was prepared. Then, the cross-linked polymer in aqueous gel phase was dried and crushed in a similar way to Example A, so that a water-absorbent resin (4) was produced.

### [Examples 5 through 8]

Examples 5 through 8 were different from Example A in that: in Example 5, acrylic acid in which 123 mass ppm had been dissolved in advance was used in place of acrylic acid of Example A, and thus would be included in a monomer to be prepared in a subsequent step; in Example 6, acrylic acid in which 615 mass ppm of hydroxyacetone had been dissolved in advance was used in place of acrylic acid of Example A, and thus would be included in a monomer to be prepared in a subsequent step; in Example 7, acrylic acid in which 1230 mass ppm of hydroxyacetone had been dissolved in advance was used in place of acrylic acid of Example A, and thus would be included in a monomer to be prepared in a subsequent step; and in Example 8, acrylic acid in which 2460 mass ppm of hydroxyacetone had been dissolved in advance was used in place of acrylic acid of Example A, and thus would be included in a monomer to be prepared in a subsequent step. In each of Examples 5 through 8, thereafter, polymerization similar to that in Example A was carried out. In Examples 5 through 8, both polymerization peak times and peak temperatures in the polymerizations were substantially similar to those in Example A. By the polymerizations, cross-linked polymers in aqueous gel phase were prepared. Then, the cross-linked polymers in aqueous phase which were dried and crushed in a similar way to Example A, so that water-absorbent resins (5) through (8) were produced in respective Examples 5 through 8.

### [Conclusion]

Results of Examples 5 through 8 and Example A are shown in Table 2.

As shown in Table 2, reductions in amount of hydroxyacetone in acrylic acid caused "enhancements in stabilities of the monomers". Regarding the water-absorbent resins which had been subjected to a color acceleration test, the reductions in amount of hydroxyacetone in acrylic acid caused decreases in YI (Yellow Index), and increases in WB (White Balance), and decreases in b values (color index). From the above, it was found that "the reduced amounts of hydroxyacetone gave the water-absorbent resins higher resistivities to the change in color over time".

**[Table 2]**

| Results of Examples 4 through 8 ··· "Effect of Hydroxyacetone" | | | | | | | |
|---|---|---|---|---|---|---|---|
| | | Example A | Example 4 | Example 5 | Example 6 | Example 7 | Example 8 |
| Additive agent | | None | Hydroxyacetone | ← | ← | ← | ← |
| Amount (of additive agent) in relation to monomer | ppm | 0 | 20 | 100 | 500 | 1000 | 2000 |
| Monomer stability | | Good | Partial | ← | Gelatinous | substance present | in degassing |
| | | | gelatification | | | | |
| | | | over long time | | | | |
| Early-phase coloring | L | 89.1 | | 90.0 | | 89.4 | |
| (immediately after production) | a | 0.0 | | -0.2 | | -0.4 | |
| | b | 3.4 | | 3.2 | | 3.9 | |
| | YI | 7.1 | | | | | |
| | WB | 75.1 | 75.1 | 75.1 | | 75.1 | |
| Coloring over time | L | 72.8 | | 72.6 | | 70.1 | |
| (after acceleration test) | a | 2.0 | | 2.0 | | 1.9 | |
| | b | 9.7 | | 10.1 | | 11.3 | |
| | YI | 26.0 | | 27.0 | | 31.0 | |
| | WB | 43.0 | 42.7 | 42.3 | | 39.1 | |

| | | | | | | | |
|---|---|---|---|---|---|---|---|
| Note: Polymerization and Physical Property in Comparative Example 1 and Examples 4 through 8 1. within the margin of error, absorbency was approximately 30 g/g, extractables were approximately 17 %, residual monomer was approximately 290 ppm, average particle diameter was 440 mm 2. within the margin of error, peak temperature in polymerization was approximately 109 °C, peak time in polymerization was approximately 15 minutes 3. Phenol was ND (not more than 1 ppm) 4. Amount of the additive agent was measured in relation to a monomer (75 mol % neutralized sodium acrylate, molar weight of 88.5) | | | | | | | |

### [Example 9]

A surface cross-linking agent including butanediol/propanediol/water = 0.32/0.5/2.73 was added to 100 weight pars of each of the water-absorbent resins (1) through (8) produced in respective Examples 1 through 8. Then, mixtures of the surface cross-linking agent and 100 weight parts of each of the water-absorbent resins (1) through (8) were heated at 210 °C for 40 minutes, so that surface cross-linked water-absorbent resins, which were referred to as water-absorbent resins (9) through (16), were produced.

### [Comparative Example 1]

Acrolein and acrylic acid were prepared in accordance with Example (paragraphs [0042] through [0053]) in International Application No. 2007/JP/68296 (Application Date: September 20, 2007). Specifically, a composition containing acrolein was purified by distillation, by using a packing column including a Dixon packing and having theoretical stages corresponding to 5 stages. The purification of the composition was carried out under a condition that a temperature in a top portion of the packing column was 46 °C, a temperature in a bottom portion of the packing column was 100 °C, a reflux ratio was 3.3, and a pressure was a normal pressure. Then, quantitative analysis of the composition by gas chromatography was carried out, and a result of the quantitative study demonstrated that 96.7 mass % of acrolein and 0.05 mass % of phenol were included in the composition. In the quantitative analysis, neither 1-hydroxyacetone, glycerin, nor allyl alcohol were detected, indicating that their amounts were less than 500 mass ppm (detection limit of 0.05 %).

Acrylic acid was prepared in accordance with Example in International Application No. 2007/JP/68296. Then, a water-absorbent resin was produced in accordance with Example in International Application No. 2007/JP/68296 (see paragraphs [0056] through [0058]).

Specifically, the monomer was prepared from unsaturated acrylic acid having a concentration of 20 mass % and temperature of 20 °C, by using 0.05 mol % of polyethylene glycol diacrylate as an internal cross-linking agent. Then, an aqueous solution of the monomer was completely polymerized in a same polymerization container as used in Examples 1 through 8. By this, a polymer in aqueous gel phase was prepared. Then, the polymer in aqueous gel phase was fragmented into pieces of approximately 1 mm, to which 62.5 g of a 48 mass % sodium hydroxide aqueous solution was then added. This carried out neutralization of 75 mol % of an acid radical of the polymer in aqueous gel phase. By this, a comparative water-absorbent resin (2) was produced.

### [Comparative Example 2]

A same water-absorbent resin as produced in Comparative Example 1 was treated by surface cross-linking in a similar way to Example 9.

### (Conclusion)

Results of Example 9 and Comparative Examples 1 and 2 are shown in Table 3. The water-absorbent resins (8) through (16), which were surface cross-linked water-absorbent resins, had same CRC, AAP (load of 50 g/cm²), and SFC. Also, the water-absorbent reins (8) through (16) had substantially same grain sizes before and after the surface cross-linking.

In Example 9, physical properties (AAP, SFC, AAP + CRC) of the water-absorbent resins (9) through (16) thus treated by the surface cross-linking were high, and decreasing rates (which were determined by AAP/CRC) of absorbencies due to a pressure were small.

**[Table 3]**

| Physical Properties After Surface Cross-Linking | | | | |
|---|---|---|---|---|
| | | Example 9 | Comparative Example 1 | Comparative Example 2 |
| Absorbency (CRC) | g/g | 32 | 50 | 40 |
| Absorbency against pressure (AAP) | g/g | 26 | 9 | 11 |
| Permeability potential (SFC) | | 30 | 0 | 2 |
| CRC + AAP | g/g | 58 | 59 | 51 |
| AAP/CRC | | 0.81 | 0.18 | 0.28 |

| | | | | |
|---|---|---|---|---|
| Note: Comparative Example 2 was originally from International Application No. 2007/JP/68296 (Application Date: September 20,2007). Comparative Example 3 was obtained by further performing, in Comparative Example 2, same cross-linking as that in Example 9. Note: unit of SFC; 10⁻⁷·sec·cm³/g | | | | |

### [Example 10]

10 g of each of water-absorbent resins (9) through (16) produced in Example 9 was uniformly mixed with 10 g of a fractured wood pulp. Then, mixtures thus prepared were pressed into a sheet form (absorbent). By use of the absorbents, disposable diapers were produced.

### [Example 11]

There was carried out same polymerization as the polymerization in Example 1 except in that an amount of Fe in NaOH was 8 mass ppm. As a result, (i) a delay in polymerization peak time occurred, thereby delaying the polymerization peak time to be 20 minutes (in Example 1, the polymerization peak time was 15 minutes), (ii) an L value was deteriorated by 1.5 point as compared to that in Example 1, and (iii) a b value was deteriorated by 0.9 as compared to that in Example 1. From this, it was clear that the Fe amount in NaOH (and the Fe amount in a monomer derived from NaOH) played an important role in control of an amount of phenol and that of hydroxyacetone.

As discussed in Examples 11 through 14, it was preferable in the present invention to use p-methoxyphenol (in amount preferably between 25 to 200 mass ppm and more preferably between 25 to 160 mass ppm) in combination with Fe (in amount preferably between 0.005 to 3 mass ppm and more preferably between 0.05 to 1 mass ppm/Fe₂O₃ basis).

### [Example 12]

There was carried out same polymerization as the polymerization in Example 1 except in that an amount of Fe in NaOH was 0.01 mass ppm. In the polymerization, a delay in polymerization onset time (induction time, 1 minutes in Example 1) occurred, thereby delaying the polymerization onset time to be 3 minutes or more. From this, it was clear that the amount of Fe in NaOH (and an Fe amount in a monomer derived from NaOH) also played an important role in control of an amount of phenol and that of hydroxyacetone.

### [Example 13]

There was carried out same polymerization as the polymerization in Example 1 except in that an amount of p-methoxyphenol was 200 mass ppm (in Example 1, 60 mass ppm). In Example 13, (i) a delay in polymerization peak time occurred, thereby delaying the polymerization peak time to be 16 minutes (in Example 1, 15 minutes), (ii) an L point was deteriorated by 5.1 point as compared to that in Example 1, and (iii) a b value was deteriorated by 2.9 point as compared to that in Example 1.

### [Example 14]

There was carried out same polymerization as the polymerization in Example 1 except in that an amount of p-methoxyphenol was 0 mass ppm (in Example 1, 60 mass ppm). In Example 14, a delay in polymerization peak time occurred, thereby delaying the polymerization peak time to be 20 minutes (in Example 1, 15 minutes). From this, it was clear that in a case where no p-methoxyphenol was used, as in the case with Patent Literatures 17 and 18, polymerization was slower.

### [Example 15]

Example A was modified in view of U.S. Patent No. 6241928 so that (i) temperature of a monomer was set at 50 °C, (ii) same degassing as that in Example A was carried out, and (iii) continuous polymerization was carried out on a belt. As a result, a continuous operation was carried out for 24 hours without interruption. In comparison of Example 15 with Example 5 in which a gelatinous material was found in a part of a pipe, it was demonstrated that control of an amount of hydroxyacetone played an important role in the continuous operation, especially in continuous operation carried out at high temperature (not lower than 50 °C).

### [Example 16]

Example 16 was same as Example 5, except in that a gel obtained after polymerization was mixed with 100 mass ppm of a chelating agent (diethylenetriamine pentaacetic acid Na). An absorbency of a water-absorbent resin was same as in Example 5, whereas the water-absorbent resin was more stable in color over time and remained whiter in color even after a color acceleration test, as compared to Example 5 (in which the water-absorbent resin was turned yellow after the color acceleration test). In addition, the water-absorbent resin had a higher anti-urine property.

### Industrial Applicability

Use of Phenol in polymerization makes a water-absorbent resin more resistible to early-phase coloring. Inclusion of Hydroxyacetone in acrylic acid ends up with a decrease in a stability of a monomer, and makes a water-absorbent resin more resistible to a change in color over time. Attention to the effects of such compounds led the inventors of the present invention to the following achievement. Namely, it was possible to produce a water-absorbent resin whiter in color even by operations such as high-temperature polymerization and drying at high temperature or polymerization of a highly-concentrated monomer, without causing either a deterioration in physical property and a decrease in productivity or a gelatification of a monomer.

## Claims

1. A method for producing a polyacrylic acid (salt)-based water-absorbent resin, the method comprising:
a preparation step in which a monomer is prepared from acrylic acid including hydroxyacetone in an amount not larger than 300 mass ppm;
a polymerization step in which the monomer is polymerized so that a hydrogel is obtained; and
a drying step in which the hydrogel is dried.

2. A method for producing a polyacrylic acid (salt)-based water-absorbent resin, the method comprising:
a preparation step in which a monomer is prepared from acrylic acid;
a polymerization step in which the monomer is polymerized in the presence of phenol so that a hydrogel is obtained; and
a drying step in which the hydrogel is dried.

3. The method as set forth in Claim 1 or 2, wherein the monomer is prepared by dissolving phenol in acrylic acid in advance.

4. The method as set forth in Claim 2 or 3, wherein the monomer includes phenol in an amount between 0.01 to 400 mass ppm based on a solid content in the monomer.

5. The method as set forth in any one of Claims 1 through 4, further comprising, after the drying step, a surface cross-linking step in which surface cross-linking of a water-absorbent resin is carried out.

6. The method as set forth in any one of Claims 1 through 5, wherein the monomer is a partially neutralized salt of acrylic acid.

7. The method as set forth in any one of Claims 1 through 6, wherein a concentration of the monomer is 35 weight % or greater.

8. The method as set forth in any one of Claims 1 through 7, wherein a temperature of the monomer is 35 °C or higher.

9. The method as set forth in any one of Claims 1 through 8, further comprising, after the drying step, a classification step in which the polyacrylic acid (salt)-based resin is classified by use of a standard sieve so that 95 weight % or more of the polyacrylic acid (salt)-based resin is a particle composition whose diameter according to the standard sieve is between 150 to 850 µm.

10. The method as set forth in any one of Claims 1 through 9, wherein acrylic acid is prepared from a material derived from a natural product.

11. The method as set forth in any one of Claims 1 through 10, wherein acrylic acid is prepared from a material derived from glycerin.

12. The method as set forth in any one of Claims 1 through 11, wherein the monomer includes a polymerization inhibitor in an amount between 25 to 200 mass % based on the monomer.

13. The method as set forth in any one of Claims 1 through 12, wherein the monomer includes iron (Fe) in an amount (Fe₂O₃ basis) between 0.01 to 1 mass ppm based on the monomer.

14. The method as set forth in any one of Claims 1 through 13, wherein in the drying step, the hydrogel is dried at drying temperature between 165 to 230 °C for drying time not longer than 50 minutes.

15. The method as set forth in any one of Claims 1 through 14, wherein in the polymerization step, continuous neutralization or continuous polymerization of the monomer is carried out.

16. The method as set forth in any one of Claims 1 through 15, wherein in the polymerization step, continuous polymerization of the monomer is carried out, and a scale of the continuous polymerization of the monomer is 1 MT/hr or greater.

17. The method as set forth in any one of Claims 1 through 16, wherein an absorbency without pressure (CRC) of the acrylic acid (salt)-based water-absorbent resin to a normal saline solution is between 10 to 45 g/g.

18. The method as set forth in any one of Claims 1 through 17, wherein the monomer further includes acetic acid and propionic acid.

19. The method as set forth in any one of Claims 1 through 18, wherein acrylic acid is prepared by a method for producing acrylic acid, which includes carrying out removal or oxidization of phenol or hydroxyacetone.

20. The method as set forth in any one of Claims 1 through 19, wherein acrylic acid for use in the preparation of the monomer contains hydroxyacetone in an amount not larger than 300 mass ppm and also includes phenol.

21. The method as set forth in any one of Claims 1 through 20, wherein acrylic acid for use in preparation of the monomer contains phenol in an amount between 0.01 to 400 ppm.

22. A polyacrylic acid (salt)-based water-absorbent resin, which (i) is a polyacrylic acid-based water-absorbent resin of which polyacrylic acid is derived from natural glycerin and (ii) has YI ≤ 30 after being subjected to a coloring acceleration test under a high-temperature and high-humidity condition.

23. The polyacrylic acid (salt)-based water-absorbent resin as set forth in Claim 22, comprising phenol.

24. The polyacrylic acid (salt)-based water-absorbent resin as set forth in Claim 22 or 23, further comprising an additive agent selected from the group consisting of a chelating agent, a reducing inorganic salt, and hydroxycarboxylic acid.

25. The polyacrylic acid (salt)-based water-absorbent resin as set forth in any one of Claims 22 through 24, further comprising acetic acid and propionic acid.

26. The polyacrylic acid (salt)-based water-absorbent resin as set forth in any one of Claims 22 through 25, wherein its absorbency against pressure (AAP) is 20 g/g or greater.

27. The polyacrylic acid (salt)-based water-absorbent resin as set forth in any one of Claims 22 through 26, wherein its absorbency without pressure (CRC) to a normal saline solution is between 10 to 45 g/g, and/or its flow conductivity (SFC) to a 0.69 % sodium chloride aqueous solution is 10 (cm³·s·10⁻⁷/g) or greater.

28. A diaper, comprising a polyacrylic acid (salt)-based water-absorbent resin as set forth in any one of Claims 22 through 27.
